# EUROPEAN PATENT APPLICATION

(11) **EP 3 381 362 A1**
(43) Date of publication of application: **03.10.2018**
(21) Application number: 17164099.8
(22) Date of filing: 31.03.2017
(51) Int. Cl.: A61B 5/055, G06T 7/00

(54) **MAGNETIC RESONANCE IMAGE QUALITY DETERMINATION**

(71) Applicant: Koninklijke Philips N.V., 5656 AE Eindhoven (NL)
(72) Inventor: KRUISKAMP, Marinus Johan, 5656 AE Eindhoven (NL)
(74) Representative: de Haan, Poul Erik

(57) **Abstract**

The invention provides for a medical instrument (100), which comprises a magnetic resonance imaging system (106) for acquiring magnetic resonance data (170) from a subject within an imaging zone (138). The medical instrument (100) further comprises a memory (162) storing a map (174, 320) defining at least one anatomical area of relevance (322, 324) and is configured to acquire magnetic resonance data (170) using the magnetic resonance imaging system (106). A magnetic resonance image (172, 300) is reconstructed using the acquired magnetic resonance data. The reconstructed image (172, 300) is matched with the map (174, 320). The at least one anatomical area of relevance (322, 324) is identified in the reconstructed image (172, 300). The quality of the reconstructed image (172, 300) is determined based on whether the magnetic resonance image (172, 300) comprises an artefact (304) located within the at least one area of relevance (322, 324) defined by the map (174, 320). A signal indicative of the validity of the reconstructed image (172) is generated, in case no artefact (304) is determined to be located within the at least one area of relevance (322, 324) defined by the map (174, 320).

## Description

### FIELD OF THE INVENTION

The invention relates to magnetic resonance imaging, in particular to the determination of quality of magnetic resonance images.

### BACKGROUND OF THE INVENTION

Magnetic resonance imaging (MRI) is a complex procedure, the result of which may depend on a plurality of parameters. Therefore, the quality of magnetic resonance images reconstructed using an MRI system is not always perfect. The quality of images may for example suffer from artefacts. There may be various reasons for artefacts occurring in a magnetic resonance image. Artifacts may for example be caused by the patient or by the MRI system.

In applications, like e.g. a magnetic resonance imaging guided radiotherapy, the quality of the magnetic resonance images is crucial and errors may have serious consequences, since the target region to which high-energy radiation is applied is defined based on such magnetic resonance images. An artifact could for example be responsible for healthy tissue being irradiated. Therefore, when artifacts occur, the acquisition of magnetic resonance data as well as the reconstruction of the magnetic resonance image have to be repeated. However, repeating the acquisition of data as well as the image reconstruction each time an artifact occurs may be time consuming and a burden for the patient.

### SUMMARY OF THE INVENTION

Various embodiments provide for a medical instrument, a method, and a computer program product as described by the subject matter of the independent claims. Advantageous embodiments are described in the dependent claims.

In one aspect, the invention relates to a medical instrument, which comprises a magnetic resonance imaging system for acquiring magnetic resonance data from a subject within an imaging zone. The medical instrument further comprises a memory, which stores machine executable instructions. The memory further stores a map defining at least one anatomical area of relevance. In addition the medical instrument comprises a processor for controlling the medical instrument. Execution of the machine executable instructions causes the processor to control the medical instrument. Magnetic resonance data is acquired using the magnetic resonance imaging system. A magnetic resonance image is reconstructed using the acquired magnetic resonance data. The reconstructed image is matched with the map defining at least one anatomical area of relevance. The at least one anatomical area of relevance is identified in the reconstructed image. The quality of the reconstructed image is determined based on whether the magnetic resonance image comprises an artefact located within the at least one area of relevance defined by the map. A signal being indicative of the validity of the reconstructed image is generated, in case no artefact is determined to be located within the at least one area of relevance defined by the map.

Embodiments may have the beneficial effect that the quality of images is judged based on the relevance of the area in which an artifact occurs. In case no artifacts are determined to be present within those anatomical areas of the image which are relevant, the signal indicative of the validity of the image is generated regardless of artifact, which may be present in areas of no relevance. Thus, a time-consuming repetition of the data acquisition, the image reconstruction as well as the quality assessment can be avoided. In addition, since the anatomical areas of relevance are free of artifacts, the risk of negative consequences for the patient, e.g. in case of a magnetic resonance imaging guided radiotherapy can be avoided.

By generating the signal indicating the validity of the reconstructed image the respective image is accepted.

In case the MRI image is generated during a radiotherapy planning or execution session, the treatment plan generation, optimization and/or execution may be continued using the valid MRI image.

According to embodiments, the map defines a plurality of anatomical areas of relevance, the areas of relevance are identified in the reconstructed image and the quality of the reconstructed image is determined based on whether the magnetic resonance image comprises an artefact located within at least one of the areas of relevance. The signal being indicative of the validity of the reconstructed image is generated, in case no artefact is determined to be located within any of the areas of relevance defined by the map.

An artefact refers to a feature appearing in a magnetic resonance image image that is not present in the original subject depicted by the image. Depending on their origin, artefacts may be classified as patient-related, signal processing-dependent and hardware (machine)-related. Artefacts are problematic, since they may affect the quality of an examination and/or be confused with pathology.

Patient-related MR artefacts comprise e.g. motion artefacts, artefacts due to flow, and metal artefacts. Motion of the patient may e.g. cause ghost images induced by periodic movements, such like cardiac or respiratory movement and blood vessel or CSF pulsation, or diffuse image noise in the phase-encoding direction induced by non-periodic movement.

Several measures are known, which may be taken in order to reduce motion artefacts, including patient immobilization, cardiac and aspiratory gating, signal suppression of the tissue causing the artefact, choosing the shorter dimension of the matrix as the phase-encoding direction, view-ordering or phase-reordering methods and swapping phase- and frequency-encoding directions to move the artefact out of the field of interest.

Flow of fully magnetized protons may e.g. influence intravascular signals due to flow enhancement or flow-related signal loss. Flow-related artefacts may e.g. be ghost images or spatial misregistration.

Metal artefacts may be caused by external or internal ferromagnetics accompanying the patient. They may introduce various quality deficits into the reconstructed images, like e.g. peripheral high signals or image distortion. For example in case of a metal implant, typically a black hole shows up at the location of the implants extending beyond the size of the implant depending on the implant's material and MRI sequences used. Countermeasures may comprise removing the external source, i.e. ferromagnetics, orientating the long axis of an implant or device parallel to the long axis of the external magnetic field, choosing an appropriate frequency encoding direction, using smaller voxel sizes, fast imaging sequences, increased readout bandwidth, avoiding gradient-echo imaging, or applying an additional gradient along the slice select gradient at the time the frequency encoding gradient is applied.

Signal processing-dependent artefacts may be caused by the ways in which the magnetic resonance data is sampled, processed and mapped out on the image matrix. Signal processing-dependent artefacts comprise e.g. chemical shift artefacts, partial volume artefacts, wrap around artefacts, and ringing artefacts.

Chemical shift artefacts, which are due to difference in resonance of protons as a result of their micromagnetic environment, may occur at the fat/water interface in the phase encoding or section-select directions.

Partial volume artefacts may arise due to the size of the voxel over which the magnetic resonance signal is averaged. Counter measurements may comprise using a smaller pixel size and/or a smaller slice thickness.

Wrap around artefacts result from a mismapping of anatomical structures that lie outside the field of view, but within the slice volume. They may be countered by applying a filter in the frequency encode direction to the acquired magnetic resonance signal to eliminate frequencies greater than the Nyquist frequency. In the phase encode direction, wrap around artefacts may be reduced by increasing the number of phase encode steps. Further, a larger field of view may be chosen. Furthermore, suppression slab (rest slabs) to dephase signals outside the field of view may also be used to suppress signals from outside the field of view.

Ringing artefacts, also referred to as Gibbs phenomenon, are caused by an under-sampling of high spatial frequencies at sharp boundaries in the MRI image. Countermeasures may comprise filtering the k-space data prior to Fourier transform, increasing the matrix size for a given field of view, the Gegenbauer reconstruction or Bayesian approach.

Machine/hardware-related artefacts may be introduced by malfunctions or incorrect adjustment of hardware components. Machine/hardware-related artefacts may e.g. comprise RF quadrature, B0 inhomogeneity, gradient field artefacts, RF inhomogeneity, asymmetrical brightness, RF noise, RF tip angle inhomogeneity, Surface coil artefacts, or slice-to-slice interference.

RF quadrature refers to RF detection circuit failures arising from improper detector channel operation. In order to correct this failure, malfunctioning components have to be fixed or replaced. External magnetic field inhomogeneity, i.e. B0 inhomogeneity, may cause spatial and/or intensity distortions. Gradient field artefacts, i.e. B1 inhomogeneity, typically cause distortions. They may be corrected by reducing the field of view, by lowering the gradient field strength or by decreasing the frequency bandwidth of radio signal. Furthermore, a repair of the malfunction of the gradient coil may be necessary. RF inhomogeneity may be due to a failure of a RF coil, a non-uniform B 1 field, or a non-uniform sensitivity of a receive coil. Asymmetrical brightness may be introduced by filters that are too tight about the signal band. RF noise like zero line is due to system noise or any cause of RF disturbance. In order to counter RF noise, the source of noise may have to be identified and removed. RF tip angle inhomogeneity may be caused by variations in RF energy required to tip protons. Bounce point artefacts may be countered by using phase-sensitive reconstruction inversion recovery techniques. Surface coil artefacts are due to RF-signal attenuation and mismatching. Slice-to-slice interference may be caused by cross-excitation of adjacent slices and may be countered by acquisition of two independent sets of gapped multi-slice images. According to embodiments, the valid reconstructed image comprises one or more artifacts located outside the at least one area of relevance defined by the map.

Embodiments may have the beneficial effect that the image does not have to be free of artifacts to be valid. Decisive for the determination of the quality of the image is not the presence or absence of artifacts, but the location of artifacts. Embodiments may thus have the advantage, that more images may be accepted as being valid and time may be saved. This is in particular beneficial for the patient, for whom e.g. a radiotherapy per se constitutes a serious burden. Furthermore, the patient may be required to stay in one position, which is difficult to achieve over a longer period of time.

According to embodiments, the valid reconstructed image comprises one or more artifacts located outside the areas of relevance defined by the map. According to embodiments, the map identifies a distribution of anatomical areas of relevance and anatomical areas of nonrelevance.

Embodiments may have the beneficial effect that the map and consequently the determination of the quality of the images may be preciously adjusted to the requirements, an image has to satisfy, in particular depending on its intended use. For example, in case of a radiotherapy, anatomical areas may comprise an area of a tumor to be irradiated as well as areas of organs at risk which must not be irradiated or which have to be protected from the radiation as far as possible.

According to embodiments, the matching of the reconstructed image with the map comprises overlaying the map and the magnetic resonance image.

Embodiments may have the beneficial effect that they allow for a simple and efficient method for matching the resonance image with the map. The map may for example comprise the contours of the anatomical area(s) of relevance. By overlaying the map over the image, an operator of the medical instrument may easily see whether there is any artifact extending into or within any area of relevance.

According to embodiments, the determining of the quality of the reconstructed image comprises marking at least one area of the magnetic resonance image comprising at least one artefact and checking, whether the at least one marked area overlaps with the at least one area of relevance defined by the map.

Embodiments may have the beneficial effect that by marking the artifact, their contours may be emphasized and it may be easier to determine whether an artifact indeed extends into an area of relevance. The marking may be performed by an operator of the medical instrument or automatically by an image analysis program, e.g. an edge detection algorithm.

According to embodiments, the a plurality of areas of the magnetic resonance image comprising each at least one artefact is marked and it is checked, whether any of the marked areas overlaps with any of the areas of relevance defined by the map.

According to embodiments, the processor further controlling the instrument to generate a signal being indicative of the invalidity of the reconstructed image, in case an artefact is determined to be located within the at least one area of relevance defined by the map.

Embodiments may have the beneficial effect, that images with artifacts in areas of relevance are invalid and maybe refused from further use. The respective image may e.g. be deleted. Thus, an efficient criterion may be provided to prevent the acceptance of an image comprising an artifact an area of relevance, which e.g. in case of a magnetic resonance imaging guided radiotherapy might lead to an incorrect adjustment of the target zone.

According to embodiments, the signal indicative of the invalidity of the reconstructed image is generated, in case an artefact is determined to be located within any of the areas of relevance defined by the map.

According to embodiments, the processor further controlling the medical instrument, in case the reconstructed image is invalid, to repeat the acquisition of magnetic resonance data, the reconstruction of an image using the acquired data, the matching with the map and the determination of the image quality.

Embodiments may have the beneficial effect, that a time-consuming repetition of the acquisition of magnetic resonance data, the reconstruction of an image using the acquired data, the matching with the map and the determination of the image quality is only performed, in case it is really necessary, i.e. an artefact occurs within one of areas of relevance. When performing a repetition of the measurement, artefact countermeasures as described above may be taken.

According to embodiments, the map is provided by a planning image provided for planning a medical treatment. Embodiments may have the beneficial effect, that the planning image may be used to define anatomical areas of relevance. The planning image may have been generated during a planning session. The planning image may be provided in form of a digital medical image of anatomical structures. A digital medical image may refer to digital medical images obtained with methods which resolve anatomical features, such as for example X-ray, computer tomography (CT), positron emission tomography (PET), single-photon emission computer tomography (SPECT), ultrasound (US) and/or magnetic resonance imaging (MRI).

The planning image may be provided by a treatment simulation/planning system for radiotherapy planning. A careful planning of an external beam radiotherapy may be necessary for ensuring that irradiation of an affected part of an organ, i.e. anatomical structure, is precisely maximized, while the impact on adjacent anatomical structures is minimized. During a planning exam comprising one or more scans, the anatomical structure that needs treatment may be identified and mapped.

According to embodiments, the map is comprised by a treatment plan defining at least one anatomical target area to be irradiated and defining the dose of radiation to be applied. Embodiments may have the beneficial effect that the treatment plan may define which areas are to be irradiated and for which areas it may be critical that they are as far as possible spared from being irradiated. According to embodiments, defines a plurality of anatomical target areas.

A treatment plan as used herein is a detailed plan which comprises operation of the radiation source for performing a radiotherapy treatment. The treatment plan defines treatment fields as well as doses to be applied to the treatment fields. The treatment plan may comprise anatomical data, e.g. in form of one or more digital medical images of anatomical structures. The treatment plan may comprise detailed instructions for the operation of the radiation source or it may simply contain a plan for irradiating various volumes of tissue with the using the radiation source. The treatment plan may further comprise a map defining areas of relevance.

During the planning scan images of the anatomical structure to be treated are generated. Those images may be processed by the treatment simulation system and transferred to a therapy planning system which may generate a treatment plan. The therapy planning system may set up the treatment fields, i.e. determining anatomical structures and sub-structures that are intended to receive the radiation. In radiation therapy, the place on the body where the radiation beam is aimed. After the simulation is finished and the images are processed, the dose of the radiotherapy may be calculated and the beam alignment designed using the TPS. The best arrangement and size of treatment fields may be defined making sure the correct amount of radiation is applied to the affected anatomical structure. Thereby the size, shape, and number of treatment fields may be determined. The treatment fields are designed to maximize the dose of radiation to the area that needs to be treated and to avoid or minimize the dose to the normal surrounding tissues. These treatment fields may provide and/or be comprised by areas of relevance. For example, areas to which a large dose is delivered maybe considered relevant, will areas to which a small or no dose is delivered may be considered non-relevant in views of the determination of the image quality as described above.

Matching the reconstructed image with the map may comprise registering the magnetic resonance image data to the treatment plan. The registration of the reconstructed image to the treatment plan may comprise identifying anatomical landmarks within the reconstructed image. The anatomical landmarks may be identified using specialized algorithms and/or rules for particular anatomical regions. For example, an edge detection algorithm may be used. For three-dimensional magnetic resonance image data, which may be provided by a stack of two dimensional cross-sections, a shape constrained deformable model or models may be used to identify anatomical landmarks.

According to embodiments, the at least one area of relevance defined by the map comprises at least one of an anatomical target area to be irradiated and an area comprising an organ at risk.

According to embodiments, the medical instrument further comprises a radiation source for emitting X-ray or gamma ray radiation directed at a target zone within the imaging zone. The processor further controlling the instrument, in case the reconstructed image is indicated to be valid, to register a location of the target zone in the reconstructed image; generate control signals in accordance with the location of the target zone and the treatment plan; and control the radiation source to irradiate the target zone using the control signals.

Radiation therapy or radiotherapy is a type of cancer treatment, where the purpose is to kill malignant cells with ionizing, high-energy radiation from a radiation source. Magnetic resonance imaging (MRI) is a medical imaging technique that enables to image the anatomy of a human body. MRI-guided radiotherapy means the use of MRI images to improve the radiation treatment deliveries.

Radiotherapy is intended to kill malignant cells, while causing minimal harm to surrounding, healthy organs. The total dose required to kill a tumor, which typically is about 20 Gy to 80 Gy, is in general distributed over multiple treatment sessions. There are two major drawbacks to be taken into account in using a radiation source for radiotherapy: the effects of the radiation on healthy tissue and the uncertainties about the location and shape of the tumor. These drawbacks establish limitations restricting the maximum radiation dose of the treatment.

Exposure to the radiation of the healthy cells surrounding a tumor may be limited by radiating the tumor from different angles and by modifying the beam shape. During a single session, further only a dose of about 2 Gy may be applied. This way healthy cells have some time to recover from the radiation before the next treatment session.

To be able to use these techniques, accurate knowledge of the tumor shape and position is required. In order to image the anatomy comprising the tumor to be treated as well as the healthy tissue surrounding the tumor, MRI may be used. The impact of the aforementioned limitations may be reduced and/or minimized by using image guided radiotherapy, e.g. based on MRI. MR-RT system refers to a magnetic resonance imaging system combined with an external radiation source for radiotherapy.

In radiotherapy, the position of the subject to be treated, i.e. the patient, is very important. The body may be positioned carefully within an imaging volume of the medical instrument in order to optimize the radiotherapy treatment to be performed. The subject may be adjusted in the same position during each treatment and have to remain still. This optimized position may be determined during the planning scan. To stabilize this position, the subject may be positioned in a special immobilization device on a treatment table of the medical instrument. Different kinds of immobilization devices may be used. Similar positioning of organs, like the prostate, at each session may be reproduced by applying predefined preparation protocols, like e.g. drinking protocols etc.

MRI may provide a superior soft-tissue contrast compared to other medical imaging techniques such as computed tomography imaging. MRI-guided radiotherapy refers to the use of MRI images to improve the radiation treatment deliveries. The possibility to use MRI images as a guidance in dose delivery planning increases the accuracy of the therapy and potentially improves the treatment outcome. While the number of diagnosed cancers keeps increasing, there is a growing need for more efficient radiation treatments. Increased accuracy of MRI-guided therapy compared to traditional radiation therapy enables the use of higher radiation doses which decreases the needed treatment session times. For MRI-guided radiotherapy a medical instrument may be used, which integrates an MRI system and a radiation source, like e.g. a linear accelerator (LINAC), used to generate the high-energy radiation. An alternative radiation source may e.g. be provided using 6°CO radionuclides. Such a medical instrument for MRI-guided radiotherapy may be used to deliver accurate radiation therapy treatments for patients with cancer.

According to embodiments, the registration of a location of the target zone in the reconstructed image may coincide with the matching of the reconstructed image with the map. For example, the map of the areas of relevance and a definition of the target zone may be provided together by the treatment plan.

According to embodiments, the location of the target zone is registered in the valid reconstructed image, i.e. the image is first indicated to be valid and only for valid images further steps are performed.

According to embodiments, the radiation source is provided by a LINAC emitting X-ray radiation. A linear particle accelerator (LINAC) is a type of particle accelerator. It increases the kinetic energy of charged subatomic particles or ions by subjecting these charged particles to a series of oscillating electric potentials along a linear beamline. LINACs are used for a variety of applications, e.g., they are used for generating X-rays for medicinal purposes in radiotherapy. Linear accelerators are in particular used in radiation treatments for patients with cancer. In radiation treatments, high-energy X-rays generated by the LINAC are used to destroy cancer cells. The radiation is generated by accelerating electrons in a waveguide and colliding the accelerated electrons to a heavy metal target. The collision produces high-energy photons in the Bremsstrahlung process. These photons are directed to the patient's tumor and shaped to conform the shape of the tumor.

According to embodiments, the radiation source comprises radionuclides emitting gamma ray radiation from gamma decays.

Here, X-rays and gamma rays are defined by their origin: X-rays are emitted by electrons, e.g. while being accelerated to produce bremsstrahlung-type radiation, while gamma rays are emitted by a nucleus.

For example, ⁶⁰CO may be used as a radiation source, which emits gamma ray radiation for medical radiotherapy. The so called cobalt therapy or cobalt-60 therapy refers to the medical use of gamma rays from the radioisotope cobalt-60 to treat conditions such as cancer. As used in radiotherapy, cobalt units produce stable, dichromatic beams of 1.17 MeV and 1.33 MeV, resulting in an average beam energy of 1.25 MeV. The cobalt-60 isotope has a half-life of 5.3 years so the cobalt-60 may needs to be replaced occasionally.

In another aspect, the invention relates to a computer program product for operating a medical instrument comprising a magnetic resonance imaging system for acquiring magnetic resonance data from a subject within an imaging zone. The computer program product comprises a computer readable storage medium having program instructions embodied therewith. The program instructions are executable by a processor of the magnetic resonance imaging system. Execution of the machine executable instructions causes the processor to control the medical instrument. A map defining at least one area of relevance is accessed. Magnetic resonance data is acquired using the magnetic resonance imaging system. A magnetic resonance image is reconstructed using the acquired magnetic resonance data. The reconstructed image is matched with the map. The at least one anatomical area of relevance is identified in the reconstructed image. The quality of the reconstructed image is determined based on whether the magnetic resonance image comprises an artefact located within the at least one area of relevance defined by the map. A signal being indicative of the validity of the reconstructed image is generated, in case no artefact is determined to be located within the at least one area of relevance defined by the map.

In another aspect, the invention relates to a method of operating a medical instrument comprising a magnetic resonance imaging system for acquiring magnetic resonance data from a subject within an imaging zone. The method comprises providing a map defining at least one anatomical area of relevance. Magnetic resonance data is acquired using the magnetic resonance imaging system. A magnetic resonance image is reconstructed using the acquired magnetic resonance data. The reconstructed image is matched with the map. The at least one anatomical area of relevance is identified in the reconstructed image. The quality of the reconstructed image is determined based on whether the magnetic resonance image comprises an artefact located within the at least one area of relevance defined by the map. A signal being indicative of the validity of the reconstructed image is generated, in case no artefact is determined to be located within the at least one area of relevance defined by the map.

As will be appreciated by one skilled in the art, aspects of the present invention may be embodied as an apparatus, a method or a computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system". Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by a plurality processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code maybe in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 shows a schematic diagram of an exemplary medical instrument for magnetic resonance imaging guided radiotherapy;
Fig. 2 shows a flow diagram of a method for determining the quality of a magnetic resonance image;
Fig. 3 shows a schematic block diagram of a valid magnetic resonance image; and
Fig. 4 shows a schematic block diagram of an invalid magnetic resonance image.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

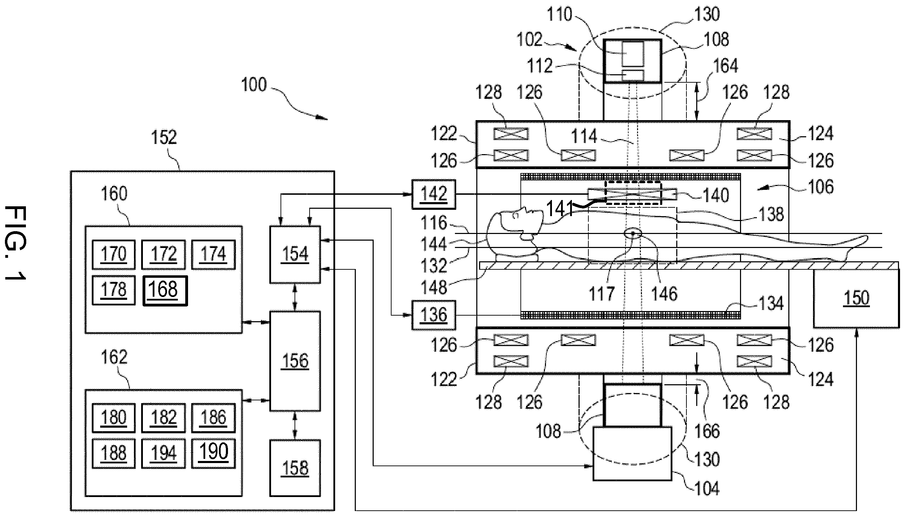

Fig. 1 shows a schematic cross-sectional and functional view of a medical instrument 100. The medical instrument 100 is shown as comprising a radiotherapy system 102 and a magnetic resonance imaging system 106. The radiotherapy system 102 comprises a ring mechanism 108. The ring mechanism 108 supports a radiotherapy source 110. The radiotherapy source 110 is representative and may, e.g., be a LINAC X-ray source, an X-ray 2 or a radioisotope gamma radiation source, like a ⁶⁰CO gamma ray source. Adjacent to the radiotherapy source 110 is a multi-leaf beam collimator 112 for collimating a radiation beam 114 that is generated by the radiotherapy source 110. The ring mechanism 108 is also adapted for moving e.g. rotating the radiotherapy source 110 and the beam collimator 112 about a rotational point 117 of the radiotherapy system 102. A rotational axis 116 passes through the rotational point 117.

The magnetic resonance imaging system 106 is shown as comprising a main magnet 122. The ring mechanism 108 is ring-shaped and surrounds the main magnet 122. The main magnet 122 shown in Fig. 1 is a cylindrical type superconducting magnet. However, other magnets are also applicable for embodiments of the invention. The main magnet 122 has a supercooled cryostat 124. Inside the cryostat 124 there is a collection of superconducting coils 126. There are also compensation coils 128 whose current opposes the direction of current in the superconducting coils 126. This creates a low magnetic field zone 130 that circles or encompasses the main magnet 122. The cylindrical main magnet 122 is shown as having an axis 132 of symmetry.

Within the bore of the magnet there is a magnetic field gradient coil 134 which is used for acquisition of magnetic resonance data to spatially encode objects within an imaging zone 138 of the main magnet 122. The magnetic field gradient coil 134 is connected to a magnetic field gradient coil power supply 136. The magnetic field gradient coil 134 is intended to be representative. Typically, magnetic field gradient coils contain three separate sets of coils for spatially encoding in three orthogonal spatial directions. The imaging zone 138 is located in the center of the main magnet 122.

Adjacent to the imaging zone 138 is a radio frequency (RF) coil 140 for manipulating the orientations of magnetic spins within the imaging zone 138 and for receiving radio transmissions from spins also within the imaging zone 138. The radio frequency coil 140 is connected to a radio frequency transceiver 142. The radio frequency coil 140 and radio frequency transceiver 142 may be replaced by separate transmit and receive coils and a separate transmitter and receiver. It is understood that the radio frequency coil 140 and the radio frequency transceiver 142 are simply representative.

The radiation beam 114 passes through the magnetic resonance imaging system 106. The radiation beam 114 in particular passes through the radio frequency coil 140. The area of the radio frequency coil 140, through which the radiation beam 114 may pass is defined by the radiation window 141.

Within the center of the main magnet 122 is also located a subject 144. The subject 144 has a target zone (or target zone) 146 and is shown as reposing on a patient carrier 148. The RF coil 140 may transmit RF pulses into the target zone 146. The patient carrier 148 has a mechanical positioning system 150. The mechanical positioning system 150 is adapted for positioning the patient carrier 148 within the main magnet 122. Depending upon the space available inside of the main magnet 122, the mechanical positioning system 150 may move the patient carrier 148 in different directions including a direction perpendicular to the magnet axis 132. If there is more space available inside the main magnet 122 the mechanical positioning system 150 may have more degrees of freedom. For instance, the mechanical positioning system 150 may position the patient carrier 148 with six degrees of freedom.

The radio frequency transceiver 142, the magnetic field gradient coil power supply 136, the mechanical actuator 104, and the mechanical positioning system 150 are all shown as being connected to a hardware interface 154 of a computer system 152. The computer system 152 uses a processor 156 to control the medical instrument 100.

The computer system 152 shown in Fig. 1 is representative. Multiple processors and computer systems may be used to represent the functionality illustrated by this single computer system 152. The computer system 152 comprises the hardware interface 154 which allows the processor 156 to send and receive messages to components of the medical instrument 100. The processor 156 is also connected to a display device 158, computer storage 160, and computer memory 162. The display device 158 may comprise a touch screen sensitive display device. The display device may be provided with a detachable stylus pen to allow a user to more efficiently manipulate the display device 158.

The radiotherapy system 102 is not shown as being connected to the hardware interface 154. The radiotherapy system 102 maybe, for example, connected to the hardware interface 154 and communicates with the computer system 152 via the mechanical actuator 104.

For the example shown in Fig. 1, the rotational axis 116 of the radiotherapy system is not coaxial with the magnet axis 132. The rotational point 117 is shown as being off center from the magnet axis 132. It can be seen that the target zone 146 is off-center and away from the magnet axis 132. The radiotherapy system 102 has been moved by mechanical actuator 104 such that the rotational point 117 of the radiotherapy system is within the target zone 146. It can be seen that the ring mechanism 108 has been moved relative to the magnet 122.

The radiation beam 114 passes through the rotational point 117. Placing the rotational point 117 at the center of the target zone 146 allows the target zone to be treated continuously when the radiation beam 114 is created by the radiotherapy source 110 and is rotated by the ring mechanism 108.

Computer storage 160 is shown as containing magnetic resonance data 170 that have been acquired by the magnetic resonance imaging system 106, using pulse sequence data 168 contained by the computer storage 160. The computer storage 160 is shown as further containing magnetic resonance images 172 that have been reconstructed from the magnetic resonance data 170. The computer storage 160 is shown as further containing a treatment plan 174. The computer storage 160 is shown as further containing radiotherapy control signals 178.

The computer memory 162 contains machine executable instructions 180, 182, 186, 188, 194 for operation by the processor 156. The computer memory 162 is shown as containing a medical instrument control module 180. The medical instrument control module 180 contains machine executable instructions which allow the processor 156 to control the overall functioning of the medical instrument 100. The computer memory 162 is shown as further containing a radiotherapy system control module 182. The radiotherapy system control module 182 contains machine executable instructions which allow the processor 156 to control the functioning of the radiotherapy system 102.

The computer memory 162 is shown as further containing a magnetic resonance imaging control module 186. The magnetic resonance imaging control module 186 contains machine executable code which allows the processor 156 to control the functioning and operation of the magnetic resonance imaging system 106. The computer memory 162 is shown as further containing an image reconstruction module 188. The image reconstruction module 188 contains machine executable code which is used by the processor 156 to transform the magnetic resonance data 170 into images 172.

The computer memory 162 further comprises an image quality module 190 containing machine executable code which enable the processor 156 to match a reconstructed image 172 with a map defining at least one anatomical area of relevance in the image 172. The map may e.g. be provided by the treatment plan 174 or in form of an additional map. Depending on whether or not the image comprises an artefact located within an area of relevance, the image is indicated to be valid or invalid for further use. According to embodiments, the image quality module 190 may further enable the processor 156 to detect and locate artefacts within the image 172.

The computer memory 162 is shown as further containing radiotherapy control signal generation module 194. The radiotherapy control signal generation module 194 contains computer executable code which the processor 156 uses to generate the radiotherapy control signals 178. The radiotherapy control signals 178 may be generated in conjunction with the treatment plan 174.

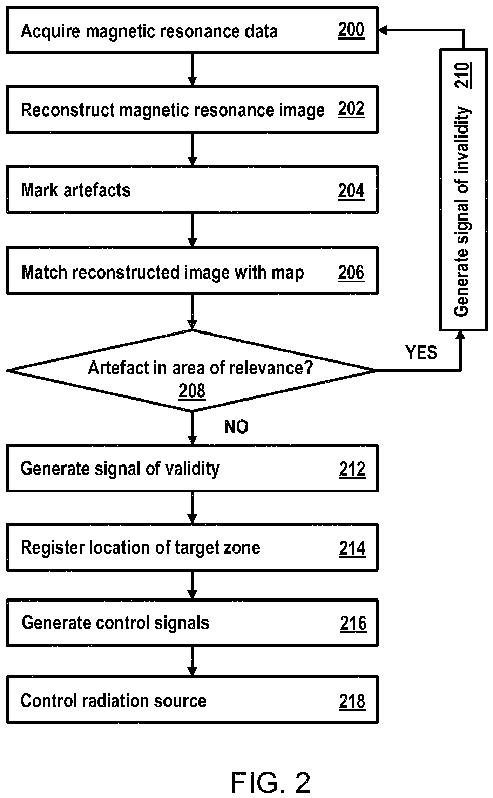

Fig. 2 shows a flow diagram of an exemplary method to determine the quality of a magnetic resonance image depending on the location of artefacts. In block 200, magnetic resonance data is acquired. The acquired data may be 2D or 3D data. In block 202, one or more magnetic resonance images are reconstructed using the magnetic resonance data acquired in block 200. Is the acquired magnetic resonance data 3D data, e.g.one or more 2D images may be reconstructed. For example, a stack of 2D cross-sections through a 3D anatomical structure may be provided. In block 204, artifacts are marked in the reconstructed images from block 202. The marking may e.g. be facultative. The marking can be performed automatically by the medical instrument using an artifacts detection and marking algorithm or by an operator of the medical instrument interacting with the instrument via an interface. The artifact detection may e.g. use an edge detection algorithm or a shape constrained deformable model. For example, anatomical landmarks are identified. In case deviations from expected general forms are detected, these detections may e.g. be identified as artefacts. In block 206, the reconstructed image is matched with the map. The map defines at least one anatomical area of relevance, which is identified in the reconstructed image. This matching may e.g. comprise overlaying of the image by the map or vice versa. According to an embodiment the matching may be performed by registering the image to the map. The registering may comprise identifying anatomical landmarks of the reconstructed image, e.g. using an edge detection algorithm or a shape constrained deformable model. In block 208, it is determined, whether there are artifacts within an anatomical area of relevance defined by the map. For example, it is determined whether one of the areas marked in block 204 overlaps with one of the areas of relevance defined by the map. This determination may be performed automatically by using an algorithm or by an operator of the medical instrument.

In case an artefact is identified in an area of relevance in block 208, the method continues with block 210. In block 210, a signal indicative of the invalidity of the image is generated. The method may continue with block 200, i.e. the data acquisition, image reconstruction, artefacts determination and quality determination according to blocks 200 to 208 is repeated. In block 212, a signal indicative of the validity of the reconstructed image is generated.

A valid image may e.g. be used for a radiotherapy. In block 214, the location of a target zone is registered to the image. The registering may comprise identifying anatomical landmarks of the reconstructed image, e.g. using an edge detection algorithm or a shape constrained deformable model. According to embodiments, block 214 can be part of block 206. The map may e.g. be provided by a treatment plan comprising information about the target zone. In block 216, control signals are generated in accordance with the location of the target zone and the treatment plan. The target zone may define the target of the radiation, the treatment plan may provide information about the dose to be applied to the target zone as well as information about the shape of the radiation beam to be applied. In block 218, the radiation source, i.e. the radiotherapy system, is controlled to irradiate the target zone according to the treatment plan using the control signals.

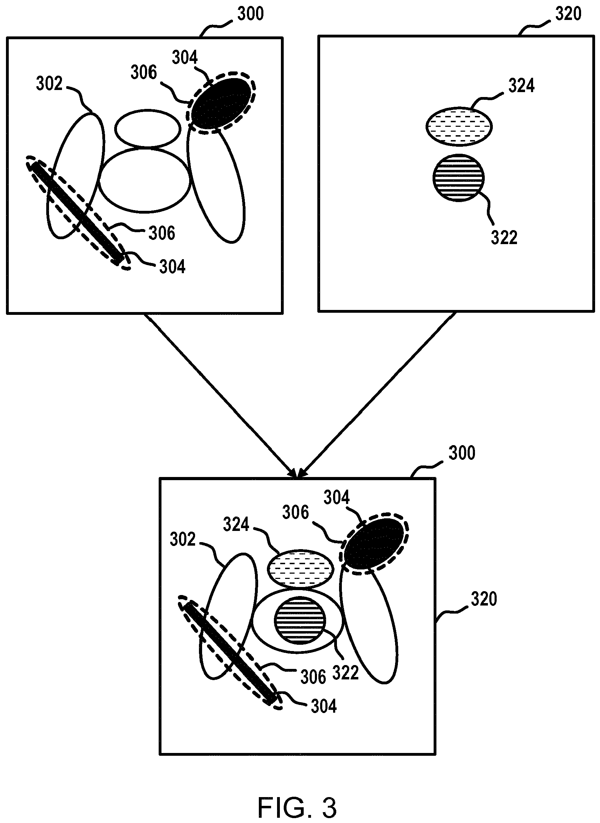

Fig. 3 shows a schematic block diagram comprising a magnetic resonance image 300. The image 300 depicts anatomical structures 302. Furthermore, the image comprises artefacts 304. The artifacts 304 have been marked by markings 306. For example, the markings are contour lines indicating areas of the image comprising one or more artefacts. Furthermore, a map 320 is provided defining areas of relevance 322, 324. According to embodiments, the map 320 may further identify areas of nonrelevance. Alternatively, all areas, which are not identified to be relevant, may be considered to be not relevant. Thus, a distribution of relevant and non-relevant areas may be provided by the map 320. Areas of relevance may for example comprise a target zone 322 to be irradiated and an organ at risk 324, which has to be prevented from being irradiated. The map 320 may e.g. be provided by a treatment plan or a planning image. The image 300 is matched with the map 320, e.g. image 300 and map 320 are overlaid with each other. Thereby, the anatomical areas of relevance 322, 324 are identified in the reconstructed image. The areas 306 with artefacts 304 do not overlap areas of relevance 322, 324. Thus, image 300 is indicated to be valid irrespectively of the artefacts 304 present in the image 300. Due to their locations, these artefacts do not spoil the quality of the image in respect of the areas of relevance.

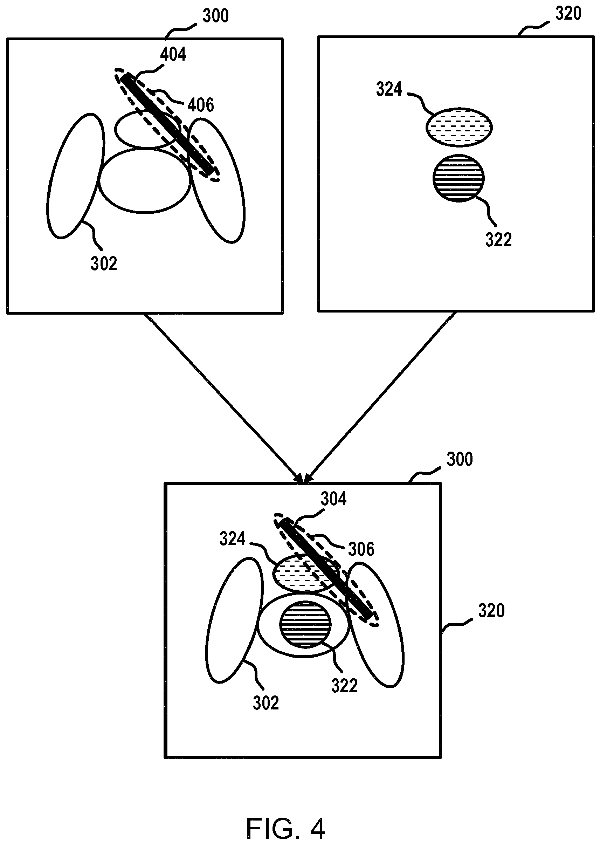

Fig. 4 shows a further schematic block diagram comprising a magnetic resonance image 300. The image 300 as well as the map 320 of Fig. 4 are identical with the image 300 and the map 320 of Fig. 3 except for the artifacts 304. The image 300 of Fig. 4 comprises only one artefact 404. The area 406 comprising the artefact 404 is marked. Overlaying the image 300 of Fig. 4 with the map 320 shows, that the artefact 404 of Fig. 4 in contrary to the artifacts 304 of Fig. 3 overlaps with area of relevance 324. Therefore, the image of Fig. 4 is indicated to be invalid due to a lack of quality. The judgment of the quality is neither dependent on the number nor on the size of the artefacts 304, 404, but rather dependent on their location. Nevertheless, a larger the number and/or size of the artefacts 304, 404 may induce a larger probability that at least one of the artefacts 304, 404 overlaps with an area of relevance. However, as long as no overlap occurs, the image quality is sufficient and the image 300 is indicated to be valid.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: radiotherapy system
- 104: mechanical actuator
- 106: magnetic resonance imaging system
- 108: ring mechanism
- 110: radiotherapy source
- 112: multi-leaf beam collimator
- 114: radiation beam
- 116: rotational axis
- 117: rotational point
- 122: main magnet
- 124: cryostat
- 126: superconducting coil
- 128: compensation coil
- 130: low magnetic field zone
- 132: magnet axis
- 134: magnetic field gradient coil
- 136: magnetic field gradient coil power supply
- 138: imaging zone
- 140: radio frequency coil
- 141: radiation window
- 142: radio frequency transceiver
- 144: subject
- 146: target zone
- 148: patient carrier
- 150: mechanical positioning system
- 152: computer system
- 154: hardware interface
- 156: processor
- 158: user interface
- 160: computer storage
- 162: computer memory
- 164: top distance
- 166: bottom distance
- 168: pulse sequence
- 170: magnetic resonance data
- 172: magnetic resonance images
- 174: treatment plan
- 178: radiotherapy control signals
- 180: medical instrument control module
- 182: radiotherapy system control module
- 186: magnetic resonance imaging control module
- 188: image reconstruction module
- 190: image quality module
- 194: radiotherapy control signal generation module
- 300: reconstructed image
- 302: anatomical structure
- 304: artefact
- 306: marking
- 320: map
- 322: target zone
- 324: organ of risk
- 404: artefact
- 406: marking

## Claims

1. A medical instrument (100) comprising a magnetic resonance imaging system (106) for acquiring magnetic resonance data (170) from a subject within an imaging zone (138), the medical instrument (100) further comprising:
- a memory (162) storing machine executable instructions, the memory (162) further storing a map (174, 320) defining at least one anatomical area of relevance (322, 324);
- a processor (156) for controlling the medical instrument (100), wherein execution of the machine executable instructions (180, 182, 186, 188, 190, 194) causes the processor (156) to control the medical instrument (100) to:
- acquire magnetic resonance data (170) using the magnetic resonance imaging system (106);
- reconstruct a magnetic resonance image (172, 300) using the acquired magnetic resonance data (170);
- match the reconstructed image (172, 300) with the map (174, 320), wherein the at least one anatomical area of relevance (322, 324) is identified in the reconstructed image (172, 300);
- determine the quality of the reconstructed image (172, 300) based on whether the magnetic resonance image (172, 300) comprises an artefact (304) located within the at least one area of relevance (322, 324) defined by the map (174, 320); and
- generate a signal being indicative of the validity of the reconstructed image (172), in case no artefact (304) is determined to be located within the at least one area of relevance (322, 324) defined by the map (174, 320).

2. The medical instrument (100) of claim 1, wherein the valid reconstructed image (172, 300) comprises one or more artifacts located outside the at least one area of relevance (322, 324) defined by the map (174, 320).

3. The medical instrument (100) of any of the previous claims, wherein the map (174, 320) identifies a distribution of anatomical areas of relevance (322, 324) and anatomical areas of nonrelevance.

4. The medical instrument (100) of any of the previous claims, wherein the matching of reconstructed image (172, 300) with the map (174, 320) comprises overlaying the map (174, 320) and the magnetic resonance image (172).

5. The medical instrument (100) of claim 4, wherein the determining of the quality of the reconstructed image (172, 300) comprises:
- marking at least one area (306) of the magnetic resonance image (172, 300) comprising artefacts (304); and
- checking, whether the at least one marked areas (306) overlaps with the at least one area of relevance (322, 324) defined by the map (174, 320).

6. The medical instrument (100) of any of the previous claims, the processor (156) further controlling the instrument (100) to generate a signal being indicative of the invalidity of the reconstructed image (172), in case an artefact (304) is determined to be located within the at least one area of relevance (322, 324) defined by the map (174, 320).

7. The medical instrument (100) of claim 6, the processor (156) further controlling the medical instrument (100), in case the reconstructed image (172, 300) is invalid, to repeat the acquisition of magnetic resonance data (170), the reconstruction of an image (172, 300) using the acquired data (170), the matching with the map (174, 320) and the determination of the image (172, 300) quality.

8. The medical instrument (100) of any of the previous claims, wherein the map (174, 320) is provided by a planning image (172, 300) provided for planning a medical treatment.

9. The medical instrument (100) of any of the previous claims, wherein the map (174, 320) is comprised by a treatment plan (174) defining at least one anatomical target area (322) to be irradiated and defining the dose of radiation to be applied.

10. The medical instrument (100) of claim 9, wherein the at least one area of relevance (322, 324) defined by the map (174, 320) comprise at least one of an anatomical target area (322) to be irradiated and an area comprising an organ at risk (324).

11. The medical instrument (100) of any of claims 9 or 10, further comprising a radiation source (110) for emitting X-ray or gamma ray radiation directed at a target zone (146) within the imaging zone (138), wherein the processor (156) further controlling the instrument (100), in response to the signal indicative of the validity of the reconstructed image (172, 300), to:
- register a location of the target zone (146) in the reconstructed image (172);
- generate control signals in accordance with the location of the target zone (146) and the treatment plan; and
- control the radiation source (110) to irradiate the target zone (146) using the control signals.

12. The medical instrument (100) of claim 11, wherein the radiation source (110) is provided by a LINAC emitting X-ray radiation.

13. The medical instrument (100) of claim 11, wherein the radiation source (110) comprises radionuclides emitting gamma ray radiation from gamma decays.

14. A computer program product for operating a medical instrument (100) comprising a magnetic resonance imaging system (106) for acquiring magnetic resonance data (170) from a subject within an imaging zone (138), the computer program product comprising a computer readable storage medium having program instructions (180, 182, 186, 188, 190, 194) embodied therewith, the program instructions (180, 182, 186, 188, 190, 194) being executable by a processor (156) of the magnetic resonance imaging system, wherein execution of the machine executable instructions (180, 182, 186, 188, 190, 194) causes the processor (156) to control the medical instrument (100) to:
- access a map (174, 320) defining at least one area of relevance (322, 324);
- acquire magnetic resonance data (170) using the magnetic resonance imaging system (106);
- reconstruct a magnetic resonance image (172, 300) using the acquired magnetic resonance data (170);
- match the reconstructed image (172, 300) with the map (174, 320), wherein the at least one anatomical area of relevance (322, 324) is identified in the reconstructed image (172, 300);
- determine the quality of the reconstructed image (172, 300) based on whether the magnetic resonance image (172, 300) comprises an artefact (304) located within the at least one area of relevance (322, 324) defined by the map (174, 320); and
- generate a signal being indicative of the validity of the reconstructed image (172), in case no artefact (304) is determined to be located within the at least one area of relevance (322, 324) defined by the map (174, 320).

15. A method of operating a medical instrument (100) comprising a magnetic resonance imaging system (106) for acquiring magnetic resonance data (170) from a subject within an imaging zone (138), the method comprising:
providing a map (174, 320) defining at least one anatomical area of relevance (322, 324);
acquiring magnetic resonance data (170) using the magnetic resonance imaging system (106);
reconstructing a magnetic resonance image (172, 300) using the acquired magnetic resonance data (170);
matching the reconstructed image (172, 300) with the map (174, 320) wherein the at least one anatomical area of relevance (322, 324) is identified in the reconstructed image (172, 300);
determining the quality of the reconstructed image (172, 300) based on whether the magnetic resonance image (172, 300) comprises an artefact (304) located within the at least one area of relevance (322, 324) defined by the map (174, 320); and
generating a signal being indicative of the validity of the reconstructed image (172), in case no artefact (304) is determined to be located within the at least one area of relevance (322, 324) defined by the map (174, 320).
